# EUROPEAN PATENT APPLICATION

(11) **EP 4 309 592 A1**
(43) Date of publication of application: **24.01.2024**
(21) Application number: 23187010.6
(22) Date of filing: 21.07.2023
(51) Int. Cl.: A61B 17/04, A61F 2/30, A61F 2/40, A61B 17/06

(54) **PROSTHESIS SUPPORTED TISSUE REPAIR SYSTEM AND METHOD**

(30) Priority: 21.07.2022 US 202263391116 P
(71) Applicant: Biomet Manufacturing, LLC, Warsaw, IN 46582 (US)
(72) Inventor: WINSLOW, Nathan A., Scottsdale, 85266 (US)
(74) Representative: Taor, Simon Edward William

(57) **Abstract**

Systems and methods for tissue repair are illustrated and described. One such system optionally includes any one or combination of: one or more sutures, a suture anchor configured to couple with the one or more sutures and a prosthesis. The prosthesis can optionally be configured to be inserted in a bone of a patient. The prosthesis can have a first surface and at least one passage therein. The at least one passage can have a first opening at the first surface and a second opening. The suture anchor can be configured to couple with the prosthesis at one or more of the first surface or the at least one passage. The one or more sutures can be configured to pass through the prosthesis via the at least one passage.

## Description

### FIELD OF THE DISCLOSURE

The present disclosure relates to deploying of a suture anchor and use of suture for tissue repair surgery that can occur during an arthroplasty procedure.

### BACKGROUND

In the human body, tissue can require repair. Such tissue includes bone, muscles, tendons, ligaments and cartilage. Forceful twisting, trauma or rotation of the knee, shoulder (or other joint) can tear or otherwise damage tissue. Disease can also necessitate replacement of bone(s) of the joint with one or more prosthetic components. Such replacement can require repair of soft tissue during the surgery. Thus, a surgical repair of the tissue may be required in various circumstances. Such repair can include suturing. Various suture assemblies have been developed for facilitating suturing and are effective for their intended purposes. Nevertheless, tissue repair assemblies for facilitating suturing are still desirable.

The human shoulder includes a rotator cuff, which is a group of tendons and muscles connecting the upper arm (humerus) to the shoulder blade (scapula). The rotator cuff tendons cover the shoulder joint and joint capsule and provide stability to the shoulder. The muscles allow the shoulder to rotate. The rotator cuff tendons also encircle the humeral head (ball) and help to keep the humeral head in the glenoid (socket) when the arm is elevated. These tendons also help to rotate the humerus on the glenoid so the arm can be raised. Without normal function of the rotator cuff, the humeral head may move upward out of the glenoid socket, which makes it difficult or impossible to raise the arm up.

A conventional or reverse joint replacement may be use used in a situation where the bone is diseased and/or the rotator cuff is damaged or lacking. This can provide some pain relief and return the shoulder joint to normal kinematic function (e.g., the patient can again raise their arm above their head).

### SUMMARY

The present disclosure provides systems including suture(s), anchor(s), prosthesis and other components. Such systems and methods that can be used to stabilize a joint including by suturing soft tissue such as the rotator cuff.

The present inventor has realized that certain aspects of arthroplasty procedures such as reattaching a sub-scapular tendon of the rotator cuff to the humerus during a shoulder replacement procedure can be overly complex and time consuming. In particular, the surgeon needs to anchor the rotator cuff back to the humerus with suture(s).

Some surgeons will anchor the rotator cuff by pre-drilling holes in the humerus. These holes can be used for suture anchor(s). This process can be done prior to resecting the proximal end of the humerus to receive a humeral prosthesis. Such drilling may be imprecise as they are dependent upon bone quality. This imprecision can result in drilling multiple holes or larger holes in the humerus, which is undesirable as the process is time consuming and removes bone unnecessarily. Alternatively, other surgeons will prepare the proximal end of the humerus to receive a humeral prosthesis but prior to implantation of the humeral prosthesis, will place suture(s) within the recess created in the humerus. These suture(s) will be anchored by the humeral prosthesis within the recess of the humerus and will extend out of a proximal end of the resected humerus adjacent the humeral prosthesis. This technique has potential drawbacks. First, the process of implanting the humeral prosthesis into the humerus can damage or cut the suture(s). If the suture(s) are damaged, this may require removal of the humeral prosthesis and replacing the suture(s). Alternatively, the suture(s) may be too loosely anchored, which can result in the suture being pulled through the bone or other complication.

The present inventor has recognized a prosthesis with one or more pre-formed passages therein. These one or more passages can be used to allow for passage of suture to a desired position on the prosthesis (and relative to the bone) with the suture anchored to the prosthesis. This configuration can save time and can reduce surgical complexity. This can result in lower surgical costs among other benefits.

Further benefits are recognized by the present inventor and can include that the hole(s) used by the suture(s) in the bone can be smaller than would be used if anchors were placed in the bone, a curved bone cutting needle may not be necessary (although it may still be used with the systems and methods discussed herein), the system can include components such as the suture(s), anchor(s), needle(s) or any one thereof that can be packaged with the prostheses and the present systems and methods eliminate the need for suture anchors to be placed in bone. Rather, the suture anchor(s) contemplated herein need only to be deployed against the prosthesis.

The above discussion is intended to provide an overview of subject matter of the present patent application. It is not intended to provide an exclusive or exhaustive explanation of the invention. The description below is included to provide further information about the present patent application

To better illustrate the systems and methods disclosed herein, a non-limiting list of examples is provided here:
Example 1 includes a system for tissue repair. The system optionally includes any one or combination of: one or more sutures, a suture anchor configured to couple with the one or more sutures and a prosthesis. The prosthesis can optionally be configured to be inserted in a bone of a patient. The prosthesis can have a first surface and at least one passage therein. The at least one passage can have a first opening at the first surface and a second opening. The suture anchor can be configured to couple with the prosthesis at one or more of the first surface or the at least one passage. The one or more sutures can be configured to pass through the prosthesis via the at least one passage.
Example 2 is the system of Example 1, optionally further comprising a guide element configured to couple with the one or more sutures at a first end thereof, wherein the first end opposes a second end of the one or more sutures that is coupled with the suture anchor, wherein the guide element is configured to facilitate passage of the one or more sutures through the at least one passage.
Example 3 is the system of Example 2, wherein optionally the guide element comprises a bone cutting needle configured to cut and pass through the bone of the patient.
Example 4 is the system of Example 3, wherein optionally the bone cutting needle is crimped to the first end of the one or more sutures.
Example 5 is the system of Example 3, wherein optionally the bone cutting needle is one of straight or curved with respect to a longitudinal axis thereof.
Example 6 is the system of any one of Examples 1-5, wherein optionally, when the prosthesis is inserted in the bone of the patient, the first surface and first opening are at a proximal side of the prosthesis and the second opening is at one of: a lateral side surface, a medial side surface, an anterior side surface, a posterior side surface or a distal side of the prosthesis.
Example 7 is the system of any one of Examples 1-6, wherein optionally the suture anchor comprises one of a non-deformable member or a deformable member having a passage therethrough, and wherein the deformable member is configured to be collapsible upon engagement with the prosthesis to form an anchoring mass having a locking profile.
Example 8 is the system of any one of Examples 1-7, wherein optionally the prosthesis comprises a humeral component of shoulder prosthesis assembly and the one or more sutures are configured to connect a rotator cuff to the bone via the suture anchor coupled to the humeral component of the shoulder prosthesis assembly.
Example 9 is the system of Example 8, wherein the humeral component of the shoulder prosthesis assembly is configured for stem-free anchoring to the bone.
Example 10 is a method of repairing tissue optionally including any one or combination of: preparing a bone facing a joint of a patient by removing a first portion of the bone, implanting a prosthesis into the bone, guiding a bone cutting instrument through the prosthesis once implanted in the bone to create a passage through a second portion of the bone, passing one or more sutures through the prosthesis and through the second portion of bone, anchoring the one or more sutures to the prosthesis and coupling the one or more sutures to a tissue of the patient.
Example 11 is the method of Example 10, wherein optionally the bone cutting instrument comprises a needle that is configured for passing the one or more sutures through the prosthesis and through the second portion of bone.
Example 12 is the method of any one of Examples 10-11, wherein optionally the second portion of the bone is distal of a proximal surface of the prosthesis and distal of a position of the anchoring of the one or more sutures to the prosthesis.
Example 13 is the method of any one of Examples 10-12, wherein optionally anchoring the one or more sutures to the prosthesis includes deforming an anchor to collapse upon engagement with the prosthesis to form an anchoring mass having a locking profile.
Example 14 is the method of any one of Examples 10-13, wherein optionally the prosthesis comprises a humeral component of shoulder prosthesis assembly and coupling the one or more sutures to the tissue includes passing the one or more sutures through a rotator cuff.
Example 15 is the method of Example 14, wherein optionally the humeral component of the shoulder prosthesis assembly is configured for stem-free anchoring to the bone.
Example 16 is a system for tissue repair. The system can optionally include any one or combination of: one or more sutures, a prosthesis, a suture anchor and a bone cutting needle. The prosthesis can optionally be configured to be inserted in a bone of a patient. The prosthesis can have at least one passage therein. The at least one passage can have a first opening on a first side of the prosthesis and a second opening at a second side of the prosthesis. The suture anchor can be configured to couple with the one or more sutures and is configured to deploy against the prosthesis. The bone cutting needle can be configured to cut and pass through the bone of the patient. The bone cutting needle can be configured to guide the one or more sutures to pass through the prosthesis via the at least one passage.
Example 17 is the system of Example 16, wherein optionally, when the prosthesis is inserted in the bone of the patient, the first side is a proximal side of the prosthesis and the second side is at one of: a lateral side surface, a medial side surface, an anterior side surface, a posterior side surface or a distal side of the prosthesis.
Example 18 is the system of any one of Examples 16-17, wherein optionally the suture anchor comprises a deformable member having a passage therethrough, and wherein the deformable member is configured to be collapsible upon engagement with the prosthesis to form an anchoring mass having a locking profile.
Example 19 is the system of any one of claims 16-18, wherein optionally the prosthesis comprises a humeral component of shoulder prosthesis assembly and the one or more sutures are configured to connect a rotator cuff to the bone via the suture anchor coupled to the humeral component of the shoulder prosthesis assembly.
Example 20 is the system of Example 19, wherein optionally the humeral component of the shoulder prosthesis assembly is configured for stem-free anchoring to the bone.
Example 21 is any one or combination of the systems and method examples above including any one or combination of the features disclosed herein.

### BRIEF DESCRIPTION OF THE FIGURES

The above-mentioned and other features and advantages of this disclosure, and the manner of attaining them, will become more apparent and the disclosure itself will be better understood by reference to the following description of examples taken in conjunction with the accompanying drawings, wherein:
FIG. 1 is an anatomic view of a shoulder joint of a patient.
FIG. 2 is a schematic diagram of a prosthesis assembly installed within the patient and forming at least a portion of the shoulder joint.
FIGS. 3-3B show a humeral implant portion of the prosthesis assembly having one or more passages therein according to an example of the present application.
FIGS. 4-4B show a humeral implant portion of the prosthesis assembly having one or more passages therein according to another example of the present application.
FIG. 5 is a schematic diagram of a system for tissue repair and/or joint replacement including one or more sutures, a guide element, a part of the prosthesis assembly and a suture anchor, according to an example of the present application.
FIG. 6 is a schematic diagram of another a system for tissue repair and/or joint replacement, according to an example of the present application.
FIGS. 7A and 7B show another example of the guide element comprising a bone cutting needle, according to an example of the present application.

Corresponding reference characters indicate corresponding parts throughout the several views. The exemplifications set out herein illustrate examples of the disclosure, and such exemplifications are not to be construed as limiting the scope of the disclosure any manner.

### DETAILED DESCRIPTION

In describing the examples of the disclosure illustrated and to be described with respect to the drawings, specific terminology will be used for the sake of clarity. However, the disclosure is not intended to be limited to any specific terms or illustrations used herein, and it is to be understood that each specific term includes all technical equivalents.

The present disclosure is directed to systems and methods that can be used in joint replacement procedures to anchor soft tissue such as ligaments. The joint replacement procedures can be total or partial procedure such as an anatomic shoulder arthroplasty or reverse shoulder arthroplasty (RSA) procedure. Although the present methods, apparatuses and systems are being described in reference to a shoulder arthroplasty, the methods, apparatuses and systems can be used for other joints such as stems for the knee, hip, ankle or the like.

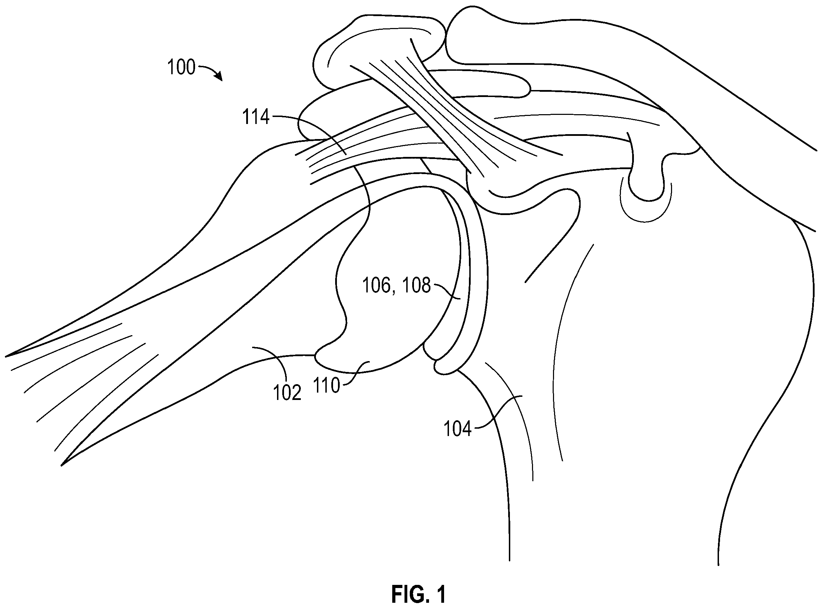

FIG. 1 illustrates a shoulder joint 100 with several ligaments stripped away. As shown, the shoulder joint 100 includes a humerus 102 and a scapula 104 that has a glenoid 106 with a socket 108 for interacting with a humeral head 110 of humerus 102. Humeral head 110 can articulate within socket 108 to allow for normal motion of the shoulder joint 100. A critical ligament for maintaining humeral head 110 within socket 108 is the rotator cuff 114. When rotator cuff 114 becomes damaged (*e.g.*, torn or degraded), normal shoulder joint function can become compromised. Since rotator cuff 114 is not functioning correctly, humeral head 110 can become dislodged from glenoid 106 during normal motion. Alternatively, disease can degenerate the bone or soft tissue of the humeral head 110 and/or scapula 104. These can cause pain and/or can negatively impact shoulder joint function. Typically, in these cases a surgical intervention may be required. Surgical intervention can repair function of the rotator cuff 114. If bone from the shoulder joint 100 must be removed, the rotator cuff 114 must be detached from the humerus 102 during the procedure and then reattached thereto. This surgical process can be complex and time consuming.

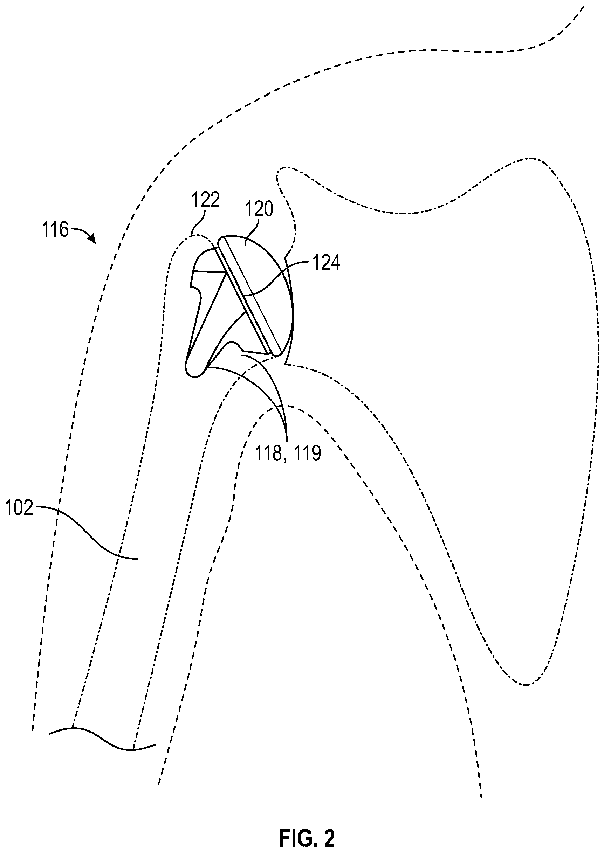

FIG. 2 illustrates a schematic diagram of a prosthesis assembly 116 installed at the shoulder joint 100. The prosthesis assembly 116 can include a humeral implant component 118 (also called a humeral component, humeral implant or humeral prosthesis component, component or similar herein) and a head component 120.

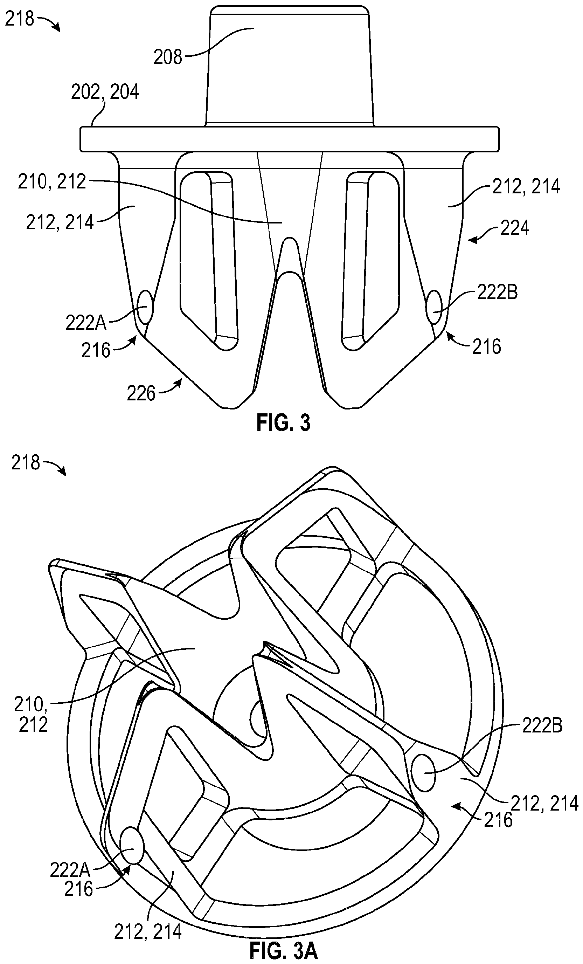

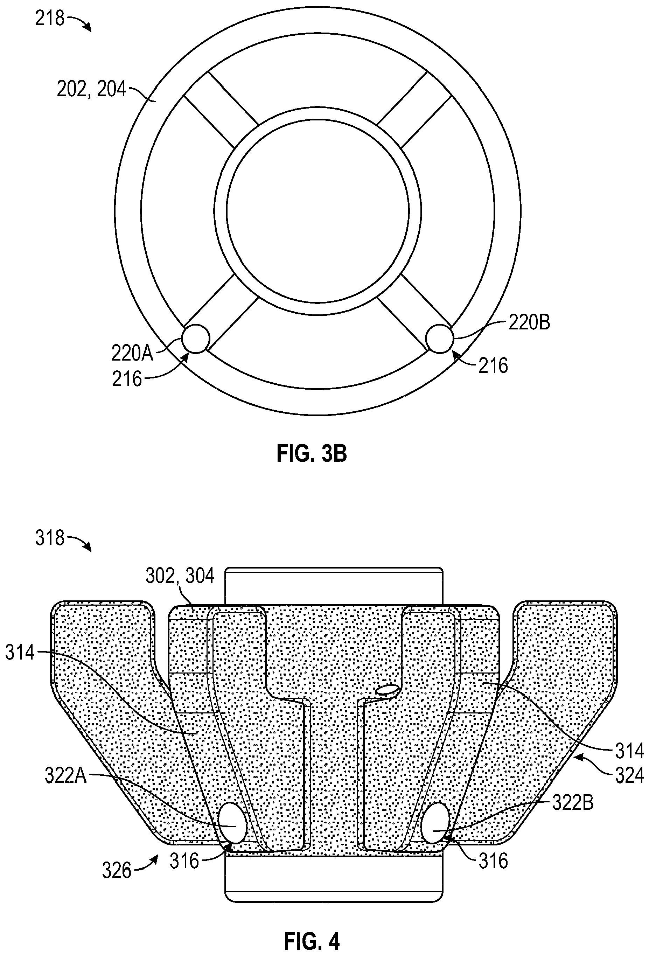

The humeral implant component 118 can be fitted into a recess 119 formed at a proximal end portion 122 of the humerus 102. The embodiment of FIG. 2 (and indeed the embodiments of FIGS. 3-4B) show a stem-less or stem-free design for the prosthesis assembly 116. Thus, the humeral implant component 118 does not couple with a stem, which would be typical in a total shoulder replacement procedure. The humeral implant component 118 without the stem can be used in a partial shoulder replacement procedure. It is contemplated that the systems and methods disclosed herein could be used with any applicable joint replacement procedure including a total shoulder replacement procedure. Thus, the concepts of the present application are not limited by the examples provided herein. Similarly, the term "bone" as used herein is not limited to the humerus but can include any applicable bone of the body including the scapula, for example.

As shown in FIG. 2, a proximal side 124 of the humeral implant component 118 can interface with the head component 120. The head component 120 can couple with the humeral implant component 118 using known mechanisms such as mating taper features or the like. The head component 120 can be semi-spherical or otherwise shaped to replicate the head of the humerus (which is removed during the joint replacement procedure).

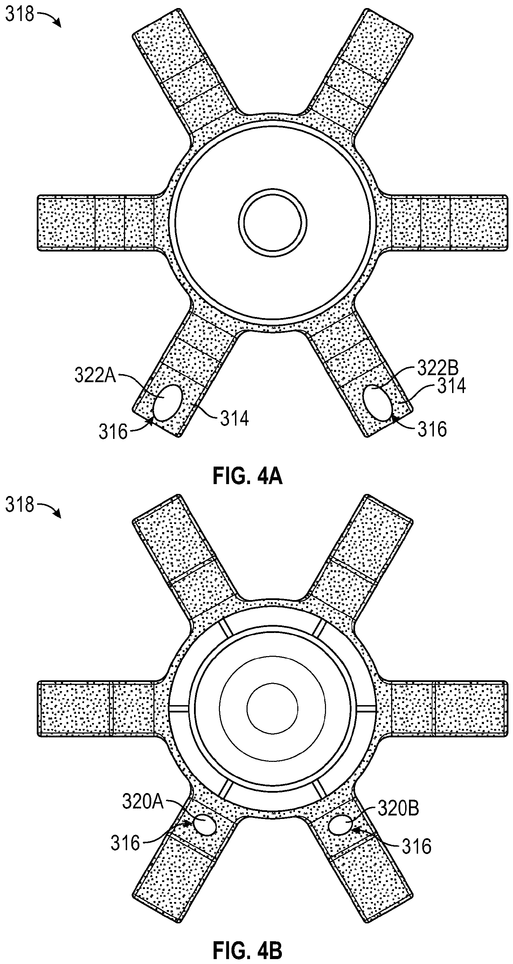

FIGS. 3-3B show an example of a humeral implant component 218. The humeral implant component 218 (and indeed, the humeral implant component 318 of FIGS. 4-4B) can be configured in the manner of the Sidus^{®} Stem-Free Shoulder prosthesis or Comprehensive^{®} Nano Stemless Shoulder commercially available and manufactured by Zimmer Biomet Inc., of Warsaw Indiana. The humeral implant component designs shown in FIGS. 2-4B are purely exemplary and are provided merely to facilitate practitioner understanding.

However, the humeral implant component 218 has been modified from the commercial products referenced above as further discussed herein. As shown in FIGS. 3, the humeral implant component 218 includes a proximal side 202 having a proximal surface 204, a coupling component 208, a body 210 and distal anchoring features 212 including fins 214. FIG. 3A shows a distal side of the humeral implant component 218 including the body 210 and the distal anchoring features 212 with fins 214. FIG. 3B shows the proximal side 202 with the proximal surface 204.

The term "proximal" refers to the general orientation of the side and/or surface when the humeral implant component 218 is implanted in the bone. Thus, "proximal" refers to a direction or location generally in the direction of or toward the head of a patient, and "distal" refers to the opposite direction of proximal, *i.e.,* away from the head of a patient. As used herein, the terms "anterior" and "posterior" should be given their generally understood anatomical interpretation. Thus, "posterior" refers to a location or direction generally toward a rear of the patient. Similarly, "anterior" refers to a location or direction generally toward a front of the patient. Thus, "posterior" refers to the opposite direction of "anterior." Similarly, the terms "medial" and "lateral" should be given their generally understood anatomical interpretation. "Medial" refers to the more inward facing (inner part) of the prosthesis (when in the implanted orientation) and "lateral" refers to the outer part or outward facing part. "Medial" refers to the opposite direction of "lateral."

As shown in FIGS. 3-3B, the humeral implant component 218 can also include one or more passages 216. The one or more passages 216 can include a first opening 220A, 220B (FIG. 3B only) and a second opening 222A, 222B (FIGS. 3 and 3A).

The first opening 220A, 220B of the one or more passages 216 can be located at the proximal side 202 in the proximal surface 204, for example. However, the first opening could be on other sides or in other components of the humeral implant component 218 according to other examples. The second opening 222A, 222B can be located at any one or combination of a lateral side surface, a medial side surface, an anterior side surface, a posterior side surface or a distal side of the humeral implant component 218. As shown in FIGS. 3 and 3A, the second opening 222A, 222B can be on both a side surface 224 and a distal side 226 of the humeral implant component 218 such as at a lower side surface of the fins 214.

The one or more passages 216 can extend through the humeral implant component 218 from the first opening 220A, 220B FIG. 3B only) to the second opening 222A, 222B (FIGS. 3 and 3A). The one or more passages 216 can be configured to direct a guide element and can receive suture(s) as further discussed herein.

The embodiment of FIGS. 3-3B shows a cementless design for the humeral implant component 218 with the coupling component 208 extending from the body 210. The proximal side 202 can be formed by interconnected fins 214. The fins 214 can be part of the anchoring features 212, which can extend from the body 210.

FIGS. 4-4B show a cemented design for the humeral implant component 318. The humeral implant component 318 can be constructed in a manner similar to that of the humeral implant component 318 but a proximal side 302 with a proximal surface 304 can be formed by part of fins 314.

As shown in FIGS. 4-4B, the humeral implant component 318 can also include one or more passages 316. The one or more passages 316 can include a first opening 320A, 320B (FIG. 4B only) and a second opening 322A, 322B (FIGS. 4 and 4A).

The first opening 320A, 320B of the one or more passages 316 can be located at the proximal side 302 in the proximal surface 304 (a top part of the fins 314), for example. However, the first opening 320A, 320B could be on other sides or in other components of the humeral implant component 318 according to other examples. The second opening 322A, 322B can be located at any one or combination of a lateral side surface, a medial side surface, an anterior side surface, a posterior side surface or a distal side of the humeral implant component 318. As shown in FIGS. 4 and 4A, the second opening 322A, 322B can be on both a side surface 324 and a distal side 326 of the humeral implant component 318 such as at a lower side surface of the fins 314.

The one or more passages 316 can extend through the humeral implant component 318 from the first opening 320A, 320B FIG. 4B only) to the second opening 322A, 322B (FIGS. 4 and 4A). The one or more passages 316 can be configured to direct a guide element (or instrument) and can receive suture(s) as further discussed herein.

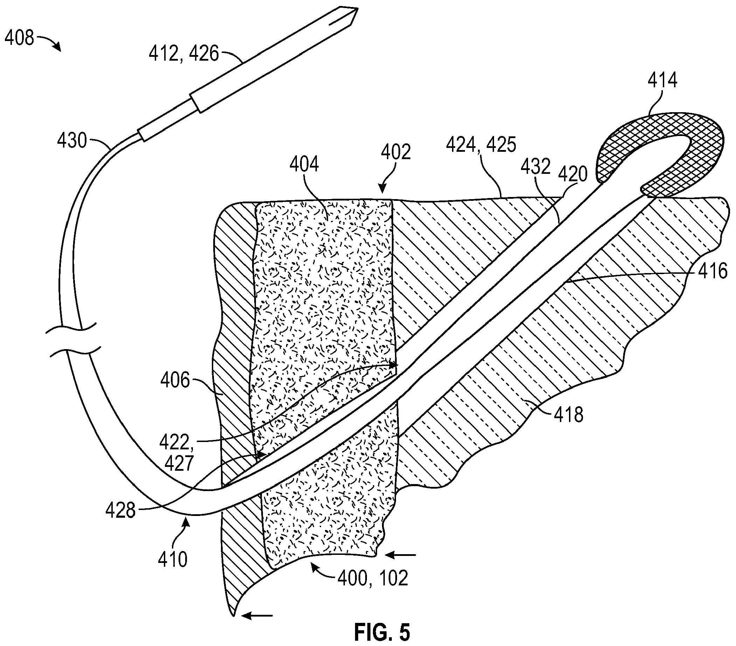

FIG. 5 shows a cross-section of a bone 400 of a patient along with a portion of a humeral implant component 418, which can be configured similar to any of the humeral implant components 118, 218 or 318, discussed previously). FIG. 5 show a proximal end 402 of the bone 400, which can comprise the humerus 102 according to one example. The bone 400 can include cancellous bone 404 and cortical bone 406.

FIG. 5 shows a system 408 for tissue repair related to the joint replacement. The system 408 can include the humeral implant component 418, one or more sutures 410, a guide element 412, and a suture anchor 414. The humeral implant component 418 can include one or more passages 416 similar to those previously discussed and illustrated in FIGS. 3-4B. The humeral implant component 418 can additionally include a first opening 420, a second opening 422 and a first surface 424 (e.g., a proximal surface when implanted into the bone 400 as illustrate in FIG. 5) at a first side 425 (e.g., a proximal side when implanted into the bone 400).

The first opening 420 of the one or more passages 416 can be at the first surface 424 as discussed with prior examples. The second opening 422 can be distal of the first opening 420 on a second side 427 of the humeral implant component 418. The second opening 422 can be one or more of lateral, medial, anterior or posterior side (at a periphery) relative to the first opening 420. Location of the first opening 420 and the second opening 422 can be as desired based upon an anatomic location of the soft tissue to be repaired with the one or more sutures 410.

The one or more passages 416 can guide the one or more sutures 410 and the guide element 412 (or alternate instrument). The guide element 412 can be configured to ease passage of the one or more sutures 410 through the one or more passages 416. Additionally, as shown in the example of FIG. 5, the guide element 412 can be configured as a bone cutting needle 426. The bone cutting needle 426 can be configured to cut the bone 400 to form a passage 428 therein. This passage 428 can have an opening distal of the proximal end 402. The passage 428 can facilitate the passage of the one or more sutures 410 from the one or more passages 416 outward from the bone 400 at a location distal of the proximal end 402 of the bone 400.

The passage 428 can be formed after implantation of the humeral implant component 418 and with the guiding aid of the one or more passages 416. Although FIG. 5 shows the guide element 412 as the bone cutting needle 426, according to other embodiments another tool or tools capable of cutting bone and/or passing suture could be utilized. For example, the guide element 412 could be a drill that can be guided through the one or more passages 416 to the bone 400 to form the passage 428. A second element such as a suture passer could then be utilized to ease or facilitate passage of the one or more sutures 410 through the one or more passages 416 and the passage 428.

The configuration of the one or more passages 416 and the passage 428 provided is purely exemplary and other angles, sizes, shapes, number, etc. are contemplated in other examples.

The one or more sutures 410 can be coupled to the bone cutting needle 426 at a first end 430 and can be coupled to the suture anchor 414 at a second end 432. The bone cutting needle 426 can be crimped or otherwise coupled to the first end 430 of the one or more sutures 410. The second end 432 may be a loop, such that a single suture can be utilized according to some examples. Multiple sutures of different sizes, types, colors and shapes are contemplated. Any type of suture as known in the art, (e.g., broadband, ribbon, round, mesh, braided, monofilament, metal, polymer, etc.) can be utilized. Thus, the system 408 can utilize multi-loaded or single-loaded suture.

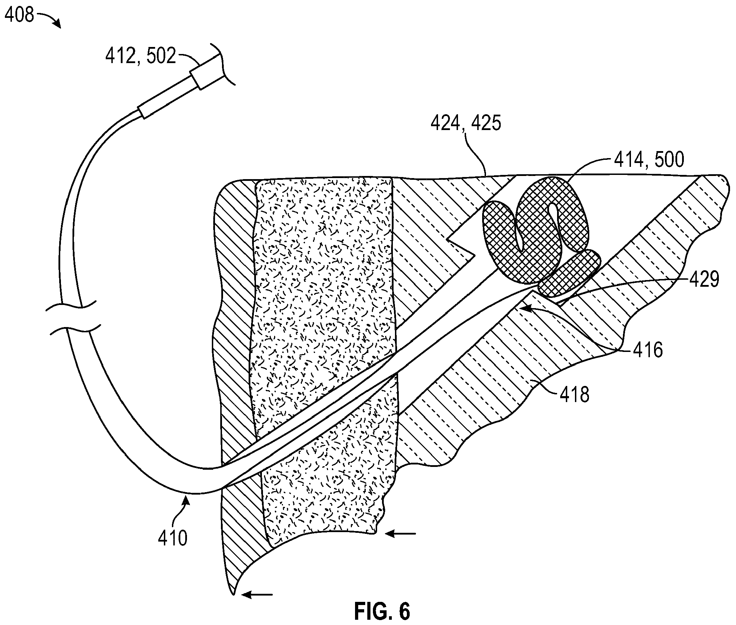

The suture anchor 414 can be of any suitable construct as known in the art. Thus, the suture anchor 414 can be hard sided so as to be substantially non-deformable upon deployment into/against the tissue of the patient (e.g., constructed as a button or another feature as known in the art). As shown in FIG. 5 the suture anchor 414 can be "soft" (e.g., they can be made from a relatively soft material such as fabric or suture), and thus they can bend, flex and/or deform under the force of a suture. In some examples, the suture anchor 414 can be nominally shaped as cylinders, or tubes having a circular or elongated cross-section with a passage therethrough. However, the suture anchor 414 can be configured to deform during deployment such as by bending (as shown in FIG. 5) knotting (as shown in FIG. 6), or otherwise changing shape. Put another way, the suture anchor 414 can comprise a deformable member having a passage therethrough. This deformable member can be configured to be collapsible upon engagement with the prosthesis to form an anchoring mass having a locking profile. As shown in FIG. 5, the locking profile of the suture anchor 414 can be configured to engage and anchor against the first surface 424 and/or parts of the one or more of the one or more passages 416. The suture anchor 414 can initially be freely placed on the first surface 424 or another location without the need for an inserter or other instrument prior to deployment of the suture anchor 414 to stabilize and hold the one or more suture 410 as shown in FIG. 5. This eliminates the need for dedicated deployment instruments for the suture anchor 414. According to the example of FIG. 5, the one or more sutures 410 and the suture anchor 414 can be a self-locking and/or adjustable loop suture assembly such as described in U.S. Patent Pub. Nos. 2014/0330311 and 2014/0046368, the disclosures of each of which is incorporated by reference herein in its entirety. Further examples of a self-locking and/or adjustable loop suture are exemplified in Applicant's ZipLoop^{®} and/or ToogleLoc^{™} Technology, which is commercially available and manufactured by Zimmer Biomet Inc., of Warsaw Indiana.

As discussed previously, it is also contemplated herein that one or more anchors utilized in the system may not be "soft" in the manner of the ZipLoop^{®}. Rather, these anchors can be made from substantially non-deformable material such as a hard plastic, metal alloy, etc. Thus, according to some examples the anchor(s) can have a cross-section or other geometry that is substantially invariant. These non-deformable anchor(s) can have a hollow interior formed by a tubular or other shape of the anchor(s).

Although a single suture anchor is illustrated in FIG. 5, in some embodiments it is contemplated that further anchors (e.g., two, three, four or more) could be used with the humeral implant component 418. The suture anchors could be differently sized/shaped relative to one another and/or can be formed of different material relative to one another according to some examples.

The system 408 include the suture anchor 414, which can be configured to couple with the one or more sutures. The humeral implant component 418 (example of a prosthesis) can be configured to be inserted in the bone 400. The suture anchor 414 can be configured to couple with the humeral implant component 418 at one or more of the first surface 424, the one or more passages 416 or another location. The one or more sutures 410 can be configured to pass through the humeral implant component 418 via the one or more passages 416. The one or more sutures 410 can be anchored to the humeral implant component 418, and hence the bone 400, via the suture anchor 414 when the suture anchor 414 is deployed against the humeral implant component 418.

To restore normal shoulder function, the one or more sutures 410 can engage with the rotator cuff (see FIG. 1) to lock the rotator cuff into position and anchor it relative to the bone 400. The one or more sutures 410 can thus provide tension and/or constraint needed during healing of the rotator cuff. The length of the one or more sutures 410 can be 12 inches to 48 inches, inclusive. However, other lengths as appropriate are contemplated.

The one or more sutures 410 can be spaced around part or the entirety of or only a portion of humeral implant component 418. A surgeon can choose to place the one or more sutures 410 along a certain location(s) and certain of the one or more passages 416 may not be utilized. A surgeon may choose to tension certain of the one or more sutures 410 (and/or one or more suture anchors 414) differently to apply an appropriate amount of tension to the soft tissue.

In operation, as discussed above, the bone 400 would be prepared to receive the humeral implant component 418 by forming a recess therein. The humeral implant component 418 would be implanted in the bone 400. Once the humeral implant component 418 is implanted, the bone cutting needle 426 would be guided by the one or more passages 416 to form the passage 428. The one or more sutures 410 would be passed through the one or more passages 416 and the passage 428 with the bone cutting needle 426. The bone cutting needle 426 can then be removed from coupling with the one or more sutures 410. The one or more sutures 410 can then be coupled to the soft tissue and tensioned as desired and this process can deploy the suture anchor 414 to anchor against the humeral implant component 418. In this manner, the soft tissue via the one or more sutures 410 can be anchored to the bone 400 (via the humeral implant component 418 engaged by the suture anchor 414).

FIG. 6 shows the system 408 including the humeral implant component 418, one or more sutures 410, the guide element 412, and the suture anchor 414. FIG. 6 differs from FIG. 5 in that the suture anchor 414 is captured by a dedicated surface, surface 429, which is not part of the first surface 424. Thus, the surface 429 can be recessed from the first surface 424 (formed by a counterbore). Although a counterbore with recessed surface 429 is shown, alternative surface(s) such as side, dedicated or projecting surfaces could be utilized for anchoring the suture anchor 414. Additionally, the suture anchor 414 of FIG. 6 is deformed into a knot structure 500 as the locking profile. According to other examples, the knot structure 500 can be at least partially engaging the surface 429 adjacent the first side 425 in addition to engaging sides of the one or more passages 416. Although not illustrated in FIGS. 5 or 6, the one or more passages 416 can be configured as desired to facilitate engagement by the suture anchor 414 (e.g., the one or more passages 416 can be provided with a restricted section, counterbore or the like). The guide element 412 can be an inserter 502 rather than a bone cutting needle. A second guide element such as a drill for forming passages in the bone is not illustrated in FIG. 6. It is contemplated that the terms "first surface" and "first side" need not be limited to a proximal surface and proximal side unless so specified. Rather, these terms can be another surface (e.g., recessed, proud, dedicated, side, distal, etc.) and can be on any side of the implant.

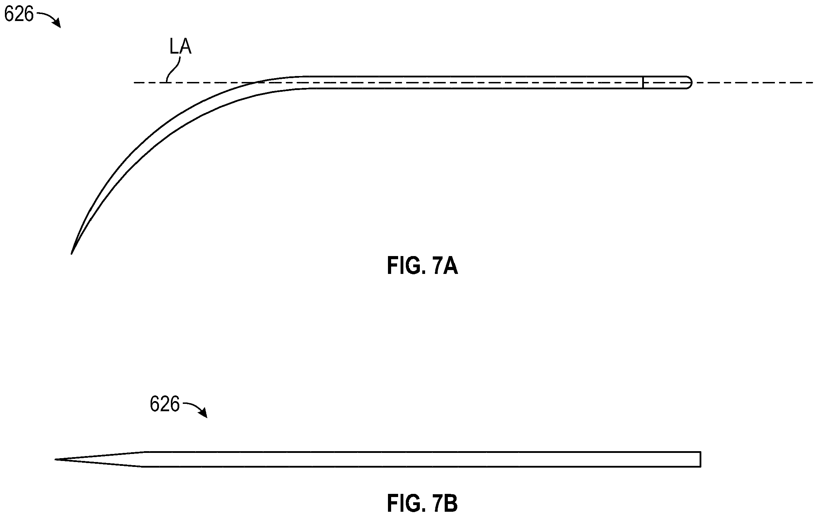

FIGS. 7A and 7B show examples of a curved bone cutting needle 626 that can be utilized according to some examples. The bone cutting needle 626 is curved relative to a longitudinal axis LA. The bone cutting needle 626 can be configured for crimping connection to the one or more sutures (not shown).

It will be readily understood to those skilled in the art that various other changes in the details, material, and arrangements of the parts and method stages which have been described and illustrated in order to explain the nature of the inventive subject matter can be made without departing from the principles and scope of the inventive subject matter as expressed in the subjoined claims. For example, the order of method steps or stages can be altered from that described above, as would be appreciated by a person of skill in the art.

It will also be appreciated that the various dependent claims, examples, and the features set forth therein can be combined in different ways than presented above and/or in the initial claims. For instance, any feature(s) from the above examples can be shared with others of the described examples, and/or a feature(s) from a particular dependent claim may be shared with another dependent or independent claim, in combinations that would be understood by a person of skill in the art.

The above detailed description includes references to the accompanying drawings, which form a part of the detailed description. The drawings show, by way of illustration, specific embodiments in which the invention can be practiced. These embodiments are also referred to herein as "examples." Such examples can include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In the event of inconsistent usages between this document and any documents so incorporated by reference, the usage in this document controls. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

The above description is intended to be illustrative, and not restrictive. For example, the above-described examples (or one or more aspects thereof) may be used in combination with each other. Other embodiments can be used, such as by one of ordinary skill in the art upon reviewing the above description. The Abstract is provided to comply with 37 C.F.R. § 1.72(b), to allow the reader to quickly ascertain the nature of the technical disclosure. It is submitted with the understanding that it will not be used to interpret or limit the scope or meaning of the claims. Also, in the above Detailed Description, various features may be grouped together to streamline the disclosure. This should not be interpreted as intending that an unclaimed disclosed feature is essential to any claim. Rather, inventive subject matter may lie in less than all features of a particular disclosed embodiment. Thus, the following claims are hereby incorporated into the Detailed Description as examples or embodiments, with each claim standing on its own as a separate embodiment, and it is contemplated that such embodiments can be combined with each other in various combinations or permutations. The scope of the invention should be determined with reference to the appended claims, along with the full scope of equivalents to which such claims are entitled.

## Claims

1. A system for tissue repair, the system comprising:
one or more sutures;
a suture anchor configured to couple with the one or more sutures; and
a prosthesis configured to be inserted in a bone of a patient, the prosthesis having a first surface and at least one passage therein, the at least one passage having a first opening at the first surface and a second opening;
wherein the suture anchor is configured to couple with the prosthesis at one or more of the first surface or the at least one passage, and wherein the one or more sutures are configured to pass through the prosthesis via the at least one passage.

2. The system of claim 1, further comprising a guide element configured to couple with the one or more sutures at a first end thereof, wherein the first end opposes a second end of the one or more sutures that is coupled with the suture anchor, wherein the guide element is configured to facilitate passage of the one or more sutures through the at least one passage.

3. The system of claim 2, wherein the guide element comprises a bone cutting needle configured to cut and pass through the bone of the patient.

4. The system of claim 3, wherein the bone cutting needle is crimped to the first end of the one or more sutures.

5. The system of claim 3, wherein the bone cutting needle is one of straight or curved with respect to a longitudinal axis thereof.

6. The system of any one of claims 1-5, wherein, when the prosthesis is inserted in the bone of the patient, the first surface and first opening are at a proximal side of the prosthesis and the second opening is at one of: a lateral side surface, a medial side surface, an anterior side surface, a posterior side surface or a distal side of the prosthesis.

7. The system of any one of claims 1-6, wherein the suture anchor comprises one of a non-deformable member or a deformable member having a passage therethrough, and wherein the deformable member is configured to be collapsible upon engagement with the prosthesis to form an anchoring mass having a locking profile.

8. The system of any one of claims 1-7, wherein the prosthesis comprises a humeral component of shoulder prosthesis assembly and the one or more sutures are configured to connect a rotator cuff to the bone via the suture anchor coupled to the humeral component of the shoulder prosthesis assembly.

9. The system of claim 8, wherein the humeral component of the shoulder prosthesis assembly is configured for stem-free anchoring to the bone.

10. A system of claim 1, wherein:
the prosthesis the at least one passage has the first opening on a first side of the prosthesis and the second opening at a second side of the prosthesis;
the suture anchor is configured to deploy against the prosthesis; and further including:
a bone cutting needle configured to cut and pass through the bone of the patient, wherein the bone cutting needle is configured to guide the one or more sutures to pass through the prosthesis via the at least one passage.

11. The system of claim 10, wherein, when the prosthesis is inserted in the bone of the patient, the first side is a proximal side of the prosthesis and the second side is at one of: a lateral side surface, a medial side surface, an anterior side surface, a posterior side surface or a distal side of the prosthesis.

12. The system of any one of claims 10-11, wherein the suture anchor comprises one of a non-deformable member or a deformable member having a passage therethrough, and wherein the deformable member is configured to be collapsible upon engagement with the prosthesis to form an anchoring mass having a locking profile.

13. The system of any one of claims 10-12, wherein the prosthesis comprises a humeral component of shoulder prosthesis assembly and the one or more sutures are configured to connect a rotator cuff to the bone via the suture anchor coupled to the humeral component of the shoulder prosthesis assembly.

14. The system of claim 13, wherein the humeral component of the shoulder prosthesis assembly is configured for stem-free anchoring to the bone.
